(19) **Europäisches Patentamt**

**European Patent Office**

**Office européen des brevets**

(11) **EP 1 301 774 B1**

(12) **EUROPÄISCHE PATENTSCHRIFT**

(45) Veröffentlichungstag und Bekanntmachung des Hinweises auf die Patenterteilung:
**12.04.2006 Patentblatt 2006/15**

(21) Anmeldenummer: **01957699.0**

(22) Anmeldetag: **29.06.2001**

(51) Int Cl.:
**G01N 21/35** (2006.01)

(86) Internationale Anmeldenummer:
**PCT/DE2001/002408**

(87) Internationale Veröffentlichungsnummer:
**WO 2002/004928 (17.01.2002 Gazette 2002/03)**

(54) **VERFAHREN ZUM NACHWEIS VON POLYNUCLEOTIDSEQUENZEN**

METHOD FOR DETECTING POLYNUCLEOTIDE SEQUENCES

PROCEDE POUR METTRE EN EVIDENCE DES SEQUENCES POLYNUCLEOTIDIQUES

(84) Benannte Vertragsstaaten:
**AT BE CH CY DE DK ES FI FR GB GR IE IT LI LU MC NL PT SE TR**

(30) Priorität: **10.07.2000 DE 10033258**
**03.11.2000 DE 10054476**

(43) Veröffentlichungstag der Anmeldung:
**16.04.2003 Patentblatt 2003/16**

(73) Patentinhaber:
 • **Haring Bolivar, Peter**
 **52146 Würselen (DE)**
 • **Büttner, Reinhard**
 **4851 Gemmernich (BE)**

(72) Erfinder:
 • **HARING BOLIVAR, Peter**
 **4730 Raeren (BE)**
 • **BÜTTNER, Reinhard**
 **4851 Gemmernich (BE)**
 • **NAGEL, Michael**
 **41751 Viersen (DE)**
 • **KURZ, Heinrich**
 **52076 Aachen (DE)**
 • **BRUCHERSEIFER, Martin**
 **Hoboken, NJ 07030 (US)**
 • **BOSSERHOFF, Katrin**
 **93053 Regensburg (DE)**

(74) Vertreter: **Bauer, Wulf**
 **Patentanwalt,**
 **Lindenallee 43**
 **50968 Köln (Marienburg) (DE)**

(56) Entgegenhaltungen:
 **WO-A-00/50867** **WO-A-99/31275**
 **WO-A-99/39008** **US-A- 5 485 277**

 • **MARKELZ A ET AL: "Pulsed terahertz spectroscopy of DNA, bovine serum albumin and collagen between 0.1 and 2.0 THz" CHEMICAL PHYSICS LETTERS, Bd. 320, Nr. 1-2, 31. März 2000 (2000-03-31), Seiten 42-48, XP001034357 in der Anmeldung erwähnt**
 • **HUNSCHE S ET AL: "THz near-field imaging" OPTICS COMMUNICATIONS, NORTH-HOLLAND PUBLISHING CO. AMSTERDAM, NL, Bd. 150, Nr. 1-6, 1. Mai 1998 (1998-05-01), Seiten 22-26, XP004127262 ISSN: 0030-4018**
 • **BRUCHERSEIFER M ET AL: "Label-free probing of the binding state of DNA by time-domain terahertz sensing" APPLIED PHYSICS LETTTERS, Bd. 77, Nr. 24, 11. Dezember 2000 (2000-12-11), Seiten 4049-4051, XP001031924**

## Beschreibung

Gegenstand der vorliegenden Erfindung ist ein Verfahren zum Nachweis einer Polynucleotidsequenz in einer Probe, welche eine Mehrzahl identischer oder verschiedener Polynucleotidsequenzen in Form von Einzelsträngen enthält, mit den Merkmalen des Oberbegriffs des Hauptanspruchs. Derartige Verfahren werden verwendet zum Nachweis bestimmter Polynucleotidsequenzen, zum Beispiel aus DNA in einer Patientenprobe.

Entsprechende Verfahren zum Nachweis bestimmter Polynucleotidsequenzen sind aus dem Stand der Technik bekannt. Das hier gattungsbestimmende Verfahren beruht auf der Verwendung einer Test-Polynucleotidsequenz, welche zur nachzuweisenden Polynucleotidsequenz komplementär ist. Kommen die zueinander komplementären, in Form von Einzelsträngen vorliegenden Polynucleotidsequenzen in Kontakt, so binden sie aneinander unter Bildung eines Doppelstrangs. Da die Bindung zwischen den Einzelsträngen im wesentlichen über Wasserstoffbrückenbindungen vermittelt werden, liegen die Energien der einzelnen Bindungen zwischen den Einzelsträngen im Bereich von Millielektronenvolt. Mittels geeigneter Nachweisverfahren wird überprüft, ob eine in der Probe enthaltenes Polynucleotidsequenz an die Test-Polynucleotidsequenzen gebunden hat oder nicht.

Die aus dem Stand der Technik vorbekannten Nachweisverfahren, welche auf diesem Nachweisprinzip beruhen, weisen im wesentlichen folgenden Verfahrensablauf auf. (siehe z.B. US-A-5 485 277)

a) Präparation einer Probe, beispielsweise einer Patientenprobe, welche eine Mehrzahl identischer oder verschiedener Polynucleotidsequenzen, hier X genannt, in Form von Einzelsträngen enthält. Diese Probe kann die nachzuweisende Polynucleotidsequenz, hier A genannt, enthalten. Dabei kann die nachzuweisende Polynucleotidsequenz A mit einer der in der Probe enthaltenen Polynucleotidsequenzen X identisch sein, oder als Baustein in einer solchen Polynucleotidsequenz X enthalten sein.

b) Präparation eines Testmediums, welches zur nachzuweisenden Polynucleotidsequenz A komplementäre Test-Polynucleotidsequenzen in Form von Einzelsträngen enthält. Im allgemeinen sind diese Test-Polynucleotidsequenzen, im folgenden mit B bezeichnet, auf einem Substrat fixiert.

c) Die Probe wird mit dem Testmedium in Kontakt gebracht, beispielsweise durch Auftropfen der in Form einer Lösung vorliegenden Probe auf das Substrat, auf welchem die komplementären Test-Polynucleotidsequenzen B fixiert sind. Unter geeigneten Bedingungen binden die eventuell in der Probe enthaltenen nachzuweisenden Polynucleotidsequenzen A an die auf dem Substrat fixierten komplementären Test-Polynucleotidsequenzen B. Eine solche Bindung zwischen den komplementären Polynucleotidsequenzen A und B ist sowohl möglich, wenn die Polynucleotidsequenz A in identischer Form in der Probe enthalten ist, als auch wenn die Polynucleotidsequenz A als Baustein einer längerkettigen Polynucleotidsequenz X in der Probe enthalten ist.

d) In einem nun folgenden Nachweisschritt wird überprüft, ob in der in Kontakt mit dem Testmedium befindlichen Probe zu einem Doppelstrang A-B verbundene Polynucleotidsequenzen A und B enthalten sind. Bei Verwendung eines Substrats, auf welchem die Test-Polynucleotidsequenzen B fixiert sind, wird hierzu der Effekt ausgenutzt, dass sich diese gepaarten komplementären Polynucleotidsequenzen A und B in stark erhöhter Konzentration an der Oberfläche des Substrats befinden. Um diese zum Doppelstrang gepaarten Polynucleotidsequenzen A-B nachzuweisen, sind aus dem Stand der Technik mehrere Nachweisverfahren bekannt.

Weite Verbreitung haben Nachweisverfahren gefunden, die auf einer Markierung der nachzuweisenden Polynucleotidsequenz A oder der hierzu komplementären Test-Polynucleotidsequenz B mit Fluoreszenzfarbstoffen beruhen. Liegen derart markierte Polynucleotidsequenzen in verdünnter Form, zum Beispiel in Lösung vor, so wird bei geeigneter Beleuchtung der Lösung nur eine schwache Fluoreszenz beobachtet. Reichert sich die markierte Polynucleotidsequenz jedoch beispielsweise aufgrund einer Anlagerung an eine komplementäre Polynucleotidsequenz, welche auf einem Substrat fixiert ist, im Bereich einer Grenzfläche an, so erhöht sich dort lokal die Dichte der fluoreszierenden Moleküle um mehrere Größenordnungen. Bei entsprechender Beleuchtung erscheinen daher diejenigen Bereiche des Substrats, an denen die nachzuweisende Polynucleotidsequenz A in erhöhtem Masse an das Substrat, genauer an die auf diesem fixierte komplementäre Test-Polynucleotidsequenz B, angelagert ist, als deutlich nachweisbar fluoreszierender Bereich der Oberfläche. Mittels quantitativer Bestimmung der Fluoreszenz, beispielsweise mittels fotografischer Aufnahme der Helligkeit der fluoreszierenden Bereiche, ist eine quantitative Bestimmung der an die komplementären Test-Polynucleotidesequenzen B gebundenen nachzuweisenden Polynucleotidsequenzen A möglich.

Entsprechende Verfahren haben mittlerweile einen hohen Entwicklungstand erreicht. Die räumlich voneinander getrennte Anordnung verschiedener bekannter komplementärer Test-Polynucleotidsequenzen B, B'... in Form eines Arrays auf einem Substrat erlauben mittlerweile den gleichzeitigen Nachweis einer Vielzahl verschiedener Polynucleotidsequenzen A, A'... in einer Probe. Entsprechende Verfahren sind beispielsweise aus [Chena 96] und [Chee 96] bekannt.

Jedoch weist die Markierung der Polynucleotidsequenzen mit Fluoreszenzfarbstoffen eine Reihe wesentlicher Nachteile auf. Einerseits bedeutet es zusätzlichen präparativen Aufwand, entweder die nachzuweisenden Polynucleotidsequenzen A oder die dazu komplementären Test-Polynucleotidsequenzen B mit geeigneten Fluoreszenzfarbstoffen zu markieren. Um die entsprechende Markierung durchführen zu können, sind biochemische Verfahren erforderlich, die einerseits mit hohem Aufwand entwickelt werden müssen und andererseits bei der großtechnischen Anwendung einen weiteren Präparationsschritt bedeuten, der zusätzliche Kosten verursacht. Weiterhin ist aus Untersuchungen bekannt, dass die Markierung von Polynucleotidsequenzen, die in Form von Einzelsträngen vorliegen, mittels Fluoreszenzfarbstoffen zu Konformationsänderungen des Polynucleotidstrangs führen können. Solche Konformationsänderungen können trotz vollständiger Komplementarität zweier einzelsträngiger Polynucleotidsequenzen zu einer verringerten Bindungswahrscheinlichkeit zwischen beiden Einzelsträngen führen, da die Konformation der Einzelstränge eine entscheidende Bedeutung für die Bindungswahrscheinlichkeit besitzt. Entsprechende Ergebnisse sind beispielsweise aus [Osaki 92] bekannt.

Weiterhin ist bekannt, dass die Markierung mit Fluoreszenzfarbstoffen die Quantifilzierbarkeit der Ergebnisse negativ beeinflusst. Dies liegt beispielsweise darin begründet, dass die Stärke der Fluoreszenz der Fluoreszenzfarbstoffe stark davon abhängt, an welcher Bindungsstelle die Fluoreszenzfarbstoffe mit der einzelsträngigen Polynucleotidsequenz verbunden sind. Weiterhin können nachfolgende Bearbeitungsschritte zu Schwankungen der Effizienz des Markierungsprozesses führen. Entsprechende Ergebnisse haben [Zhu 94], [Zhu 97] und [Laramendi 98] präsentiert.

Aus diesen Gründen wäre es wünschenswert, ein im Rahmen des gattungsgemäßen Verfahrens einsetzbares Nachweisverfahren für das Vorliegen von in Form eines Doppelstrangs miteinander verbundener Nucleotidsequenzen A und B zu finden, welches keine zusätzlichen präparativen Schritte erforderlich macht und keine negativen Auswirkungen auf die Gewinnung quantitativer Ergebnisse zeigt.

Ein im Vergleich zu der Markierung mit Fluoreszenzfarbstoffen eher physikalischer ausgerichteter Ansatz ist die Untersuchung von einzelsträngig oder in gepaarter Form vorliegenden Polynucleotidsequenzen A und B, die an eine Grenzfläche angelagert sind, mittels spektroskopischer Methoden. So wurde beispielsweise die Methode der Forier-Transformation-Infrarotspektroskopie (kurz FTIR) zur Unterscheidung zwischen einzelsträngig und doppelsträngig vorliegenden Polynucleotidsequenzen vorgeschlagen. Jedoch zeigten die Ergebnisse nur eine schwache Abhängigkeit von der eigentlich relevanten Frage, nämlich, ob die komplementären Polynucleotidsequenzen A und B in Form von Einzelsträngen oder in gepaarter Form vorlagen. Bislang ist nicht zu erkennen, dass mittels der Methode der FTIR ein zuverlässiger und quantitativer Nachweis des Bindungszustands einer nachzuweisenden Polynucleotidsequenz A an eine komplementäre Polynucleotidsequenz B möglich würde.

An dieser Stelle setzt nun die Erfindung ein. Sie hat es sich zur Aufgabe gemacht, das gattungsgemäße Verfahren so weiterzuentwickeln, dass auf eine Markierung der nachzuweisenden Polynucleotidsequenz A oder der dazu komplementären Polynucleotidsequenz B, z. B. mittels Fluoreszenzfarbstoffen verzichtet werden kann. Gleichzeitig soll gegenüber dem vorbekannten Verfahren, welches auf der Verwendung eines FTIR-basierten Nachweisverfahrens des Bindungszustands der Polynucleotidsequenzen A und B basiert, eine deutlich erhöhte Empfindlichkeit erreicht werden, die einen Nachweis des Bindungszustands zwischen den komplementären Polynucleotidsequenzen A und B ermöglicht.

Insbesondere soll das gattungsgemäße Nachweisverfahren auch zur quantitativen Bestimmung der Konzentration der nachzuweisenden Polynucleotidsequenz A in der Probe verwendet werden können.

Gelöst wird diese Aufgabe durch ein Verfahren mit den Merkmalen des Hauptanspruchs.

Es handelt sich somit um ein Verfahren zum Nachweis einer Polynucleotidsequenz A in einer Probe, wobei die Polynucleotidsequenz A in der Probe (auch) in Form von Einzelsträngen vorliegt. Die Probe enthält eine Mehrzahl identischer oder verschiedener Polynucleotidsequenzen X (auch) in Form von Einzelsträngen. Die nachzuweisende Polynucleotidsequenz A kann dabei mit einer der Polynucleotidsequenzen X identisch sein oder kann in einer der Polynucleotidsequenzen X als Sequenzabschnitt enthalten sein.

Das Nachweisverfahren für die Polynucleotidsequenz A beruht auf der Bindung der nachzuweisenden Polynucleotidsequenz A an eine bekannte, zur Polynucleotidsequenz A komplementäre Test-Polynucleotidsequenz B und an einer Abfrage des Bindungszustands, welcher zwischen den in der Probe enthaltenen Polynucleotidsequenzen X und der Test-Polynucleotidsequenz B vorliegt. Dabei weist das Verfahren die folgenden Verfahrensschritte auf:

a) Präparation eines Testmediums, welches zur nachzuweisenden Polynucleotidsequenz A komplementäre Test-Polynucleotidsequenzen B in Form von Einzelsträngen enthält,

b) In Kontakt bringen der Probe mit dem Testmedium durch Ein- oder Auf bringen der Probe in oder auf das Testmedium, so dass die in der Probe enthaltenen Einzelstränge der Polynucleotidsequenzen X an die im Testmedium enthaltenen komplementären Test-Polynucleotidsequenzen B binden können. Dabei wird eine Bindung zwischen einer bestimmten Polynucleotidsequenz X und der Test-Polynucleotidsequenz B im wesentlichen dann auftreten, wenn die bestimmte Polynucleotidsequenz X mit der nachzuweisenden Polynucleotidsequenz A identisch ist oder die nachzuweisende Polynucleotidsequenz A als Sequenzabschnitt enthält. Aufgrund der Komplementarität der Polynucleotidsequenzen A und B tritt dann eine Bindung auf.

c) Abfrage des Bindungszustandes von Polynucleotidsequenzen X an Test-Polynucleotidsequenzen B durch Ermittlung zumindest einer Komponente des komplexen Brechungsindexes oder einer äquivalenten Größe der mit dem Testmedium in Kontakt befindlichen Probe. Dies geschieht durch Wechselwirkung der in Kontakt mit dem Testmedium befindlichen Probe mit einfallender elektromagnetischer Strahlung, deren Frequenz im Bereich zwischen 0,1 Terahertz (THz) und 20 THz, vorzugsweise zwischen 1 THz und 10 THz liegt. Nach der Wechselwirkung der elektromagnetischen Strahlung mit der in Kontakt mit dem Testmedium befindlichen Probe werden die Eigenschaften der elektromagnetischen Strahlung analysiert, insbesondere im Hinblick auf Auftreten einer Zeit- oder Phasenverzögerung, einer Absorption, einer Refraktion oder Dispersion der einfallenden elektromagnetischen Strahlung verursacht durch die Wechselwirkung mit der in Kontakt mit dem Testmedium befindlichen Probe.

Da eine Bindung zwischen einer Polynucleotidsequenz X und der Test-Polynucleotidsequenz B im wesentlichen nur dann auftritt, wenn die Test-Polynucleotidsequenz X mit der nachzuweisenden Polynucleotidsequenz A identisch ist oder diese in identischer Form enthält, ist der Nachweis einer Bindung von Polynucleotidsequenzen X an die Test-Polynucleotidsequenz B ein Nachweis dafür, dass die Probe die nachzuweisende Polynucleotidsequenz A entweder in identischer Form enthält oder als Abschnitt einer längerkettigen Polynucleotidsequenz X.

Das erfindungsgemäße Verfahren umfasst daher einen Verfahrensschritt (c, der zum Nachweis des Bindungszustands der Test-Polynucleotidsequenz B dient, d.h. zur Abfrage, ob die Test-Polynucleotidsequenz in denaturierter oder in hybridisierter vorliegt. Dieser Verfahrensschritt c) beruht auf der Wechselwirkung der in Kontakt mit dem Testmedium befindlichen Probe mit elektromagnetischer Strahlung im Terahertzbereich und der Erfassung mindestens einer Komponente des komplexen Brechungsindexes der in Kontakt mit dem Testmedium befindlichen Probe oder einer dazu äquivalenten Größe wie z.B. Absorption, Transmission oder Reflexion.

In einer ersten Weiterbildung des erfindungsgemäßen Verfahren wird in einem nachfolgenden Verfahrensschritt die in Schritt c) ermittelte Komponente des komplexen Brechungsindexes oder die dazu äquivalente Größe mit einem Referenzwert verglichen. Anhand des Vergleichs mit dem Referenzwert ist zumindest eine qualitative, oftmals aber auch eine quantitative Aussage bezüglich des Vorliegens einer Bindung zwischen Polynucleotidsequenzen X und der Test-Polynucleotidsequenz B möglich.

Im Rahmen von Vorversuchen hat es sich als vorteilhaft herausgestellt, den Realteil des komplexen Brechungsindexes der mit dem Testmedium in Kontakt befindlichen Probe als den Bindungszustand kennzeichnende Größe heranzuziehen. Beim Übergang der nachzuweisenden Polynucleotidsequenz A von der in der Probe vorliegenden Einzelstrangform und der im Testmedium in Einzelstrangform vorliegenden komplementären Test-Polynucleotidsequenz B zu einem miteinander verbundenen Doppelstrang A-B tritt eine Brechungsindexänderung im THz-Bereich der in Kontakt mit dem Testmedium befindlichen Probe auf, die mittels aus dem Stand der Technik bekannter experimenteller Methoden reproduzierbar auflösbar ist. Daher kann der Realteil des Brechungsindexes als Nachweis für das Vorliegen einer Bindung zwischen der nachzuweisenden Polynucleotidsequenz A und der dazu komplementären Test-Polynucleotidsequenz B verwendet werden. Insbesondere kann dies durch Vergleich mit einem Referenzwert, der sich z. B. auf ungepaarte Test-Polynucleotidsequenzen B bezieht, geschehen.

Eine Reihe theoretischer Arbeiten beschäftigt sich mit den Anregungszuständen doppelsträngiger Polynucleotidsequenzen, siehe hierzu beispielsweise [Zhuang 90], [Young 90] und [Feng 91]. Diese sagen eine Vielzahl möglicher Anregungszustände einzel- und doppelsträngiger Polynucleotidsequenzen, beispielsweise in Form von Vibrationsmoden etc. voraus. Dabei tritt eine Vielzahl möglicher Anregungszustände erst in dem Moment auf, in dem sich zwei einzelsträngige Polynucleotidsequenzen zu einem Doppelstrang binden. Ebenso ist es möglich, dass bestimmte Anregungszustände der Einzelstränge nicht mehr auftreten oder stark unterdrückt sind, wenn sie zu einem Doppelstrang binden. Die Energiebereiche, in denen Anregungen der getrennt vorliegenden Einzelstränge auftreten, ist deutlich verschieden von dem Energiebereich, in dem Anregungszustände auftreten, die auf das Vorliegen einer doppelsträngigen Polynucleotidsequenz zurückzuführen sind.

Trotz dieser Aussagen, die sich aus den theoretischen Vorarbeiten ergeben, ist bislang eine konkrete Angabe des Energiebereichs, in welchem diese für das Vorliegen einer doppelsträngigen Polynucleotidsequenz typischen Anregungen auftreten, nicht möglich. Es hat sich im Rahmen experimenteller Untersuchungen überraschend gezeigt, dass das Abfragen des Bindungszustandes zwischen zwei komplementären einzelsträngigen Polynucleotidsequenzen A und B mit hoher Effektivität in dem im Hauptanspruch genannten Frequenzintervall möglich ist.

Aufgrund der theoretischen Arbeiten zu den Anregungszuständen gepaart vorliegender Polynucleotidsequenzen lässt sich die Hypothese aufstellen, dass mittels der Bestimmung mindestens einer Komponente des komplexen Brechungsindexes in dem im Hauptanspruch genannten Frequenzintervall eben diejenigen Anregungszustände der doppelsträngig vorliegenden Polynucleotidsequenz abgefragt werden, welche im wesentlichen durch die Doppelstrang-Konformation der Polynucleotidsequenz verursacht werden. Eine Optimierung des erfindungsgemäßen Nachweisverfahrens sollte daher möglich sein, indem die mindestens eine Komponente des komplexen Brechungsindexes der in Kontakt mit dem Testmedium befindlichen Probe in einem solchen Frequenzbereich erfasst wird, in welchem Anregungszustände entweder nur der in denaturierter, das heißt in doppelsträngiger Form vorliegenden Polynucleotidsequenz oder nur der in einzelsträngiger Form vorliegenden Polynucleotidsequenz auftreten, die im wesentlichen auf die Doppelstrangstruktur bzw. Einzelstrangstruktur zurückzuführen ist.

Besondere Vorteile bei der Durchführung des erfindungsgemäßen Verfahrens ergeben sich, wenn die Ermittlung der mindestens einen Komponente des komplexen Brechungsindexes oder der dazu äquivalenten Größe gemäß Verfahrensschritt c) des Hauptanspruchs mittels Terahertzspektroskopie (TS) erfasst wird.

Insbesondere hat es sich im Rahmen von Vorversuchen als besonders vorteilhaft herausgestellt, wenn die zur Terahertzspektroskopie verwendete Strahlung gepulst ist, das heißt, die Erfassung mittels gepulster Terahertzspektroskopie (PTS) durchgeführt wird. Besondere Vorteile ergeben sich hierbei, wenn die verwendeten Terahertzpulse eine Pulsdauer zwischen 0,05 Picosekunden und 10 Picosekunden aufweisen. Um mindestens eine Komponente des komplexen Brechungsindexes oder einer dazu äquivalenten Größe im Rahmen der PTS zu erfassen, werden im Rahmen dieses Verfahrens die Pulse nach ihrer Wechselwirkung mit der in Kontakt mit dem Testmedium befindlichen Probe zeitaufgelöst detektiert. Die zur gepulsten Terahertzspektroskopie erforderlichen Techniken sind aus dem Stand der Technik bekannt. Die grundlegenden Techniken und Methoden sind beispielsweise in [Smith 88], [Nuss 98] und [Haring 99] beschrieben. Weitere Informationen zur zeitaufgelösten PTS können auch [Wittlin 95] entnommen werden.

Die Generierung der erforderlichen gepulsten elektromagnetischen Strahlung in dem im Hauptanspruch angegebenen Frequenzbereich mit zur Durchführung zeitaufgelöster Messungen geeigneter Pulsdauer kann beispielsweise mittels in Halbleitertechnik aufgebauten fotoleitenden Schaltern (photoconductive switches) geschehen. Zur Generierung der Pulse im Terahertzbereich werden diese fotoleitenden Schalter mittels ultrakurzer Laserpulse, deren Dauer im Bereich von einigen 10 Femtosekunden liegt, und deren Wellenlänge typisch im Bereich des nahen Infraroten liegt, hier insbesondere zwischen 700 und 900 Nanometern, bestrahlt. Die zugrundeliegenden Mechanismen können beispielsweise [Katzenellenbogen 92] entnommen werden. Insbesondere können zur Verbesserung der Ausstrahlungscharakteristik der Terahertzstrahlung geeignete Antennen verwendet werden, die in der Nähe des fotokonduktiven Schalters angeordnet werden und die Abstrahlungseigenschaften der THz-Quelle verbessern.

Der grundsätzliche Aufbau eines Systems zur Durchführung der zeitaufgelösten PTS kann beispielsweise [Cai 98] entnommen werden.

Grundsätzlich wird die zeitaufgelöste PTS wie folgt durchgeführt:

1. Generierung gepulster elektromagnetischer Strahlung, deren Frequenz im Terahertzbereich liegt, und deren Pulsdauer typisch 0,5 bis 5 Picosekunden beträgt. Insbesondere werden Pulse verwendet, deren Frequenzbreite etwa 1 bis 5 Gigahertz beträgt.

2. Die gepulste Terahertzstrahlung wird mittels geeigneter Strahlführungseinrichtungen zur Probe geführt.

3. Die gepulste Terahertzstrahlung wird in Wechselwirkung mit der zu untersuchenden Probe gebracht, die sich in Kontakt mit dem Testmedium befindet.

4. Nach der Wechselwirkung mit der Probe wird die gepulste Terahertzstrahlung mittels geeigneter Strahlführungseinrichtungen zu einem Detektor geleitet.

5. Im Detektor wird die einfallende gepulste Terahertzstrahlung, welche mit der Probe in Wechselwirkung gestanden hat, analysiert. Insbesondere wird der zeitliche Verlauf der im Detektor einfallenden Pulse zeitaufgelöst erfasst. Diese zeitaufgelöste Erfassung erfolgt vorzugsweise relativ zu Terahertzpulsen, welche nicht mit der Probe in Wechselwirkung gekommen sind.

Zur zeitaufgelösten Erfassung der Terahertzpulse nach der Wechselwirkung mit der Probe sind zwei verschiedene Verfahren bekannt. Ein erstes Verfahren beruht auf der Veränderung der optischen Eigenschaften eines elektrooptischen Kristalls, welcher vom Terahertzpuls durchlaufen wird. Diese Veränderung der optischen Eigenschaften des elektrooptischen Kristalls werden mittels eines zeitlich gesehen kürzeren Pulses im Bereich des sichtbaren Spektrums oder des nahen Infrarot, insbesondere mittels Femtosekundenpulsen im nahen Infrarot, zeitaufgelöst abgefragt.

In einem alternativen Zugang werden die Änderungen der Leitfähigkeit eines wiederum in Halbleitertechnik ausgeführten fotokonduktiven Schalters als Folge einfallenden Terahertzpulse auf rein elektronischem Wege zeitaufgelöst abgefragt. Beide Verfahren sind zueinander äquivalent und können im Rahmen des hier beanspruchten Verfahrens alternativ verwendet werden. Einzelheiten zur zweiten Methode können beispielsweise [Dahl 1998] entnommen werden.

Mittels geeigneter Fouriertransformationsverfahren kann aus dem zeitaufgelösten Verlauf der Terahertzpulse, welche mit der Probe in Wechselwirkung gestanden haben, beispielsweise der Intensitätsverlauf der Terahertzpulse als Funktion der Frequenz ermittelt werden. Durch Vergleich des Intensitätsverlaufs eines Pulses, welcher mit der Probe in Wechselwirkung gestanden hat, mit dem Intensitätsverlauf eines Referenzpulses, welcher nicht mit einer Probe in Wechselwirkung war, kann direkt die (frequenzabhängige) Transmission der Terahertzpulse durch die Probe erfasst werden. Auf diese Weise kann die Transmission durch eine zu untersuchende Probe mit der Transmission durch eine Probe verglichen werden, die sicher keine Moleküle des nachzuweisenden Polynucleotids A enthält. Eine eventuell auftretende Schwächung oder Zunahme der Intensität der durch die Probe transmittierten Terahertzpulse relativ zur Referenz kann damit direkt als Nachweis dafür herangezogen werden, ob die in der Probe, welche sich in Kontakt mit dem Testmedium befindet, enthaltenen Polynucleotidsequenzen X in Gegenwart der Test-Polynucleotidsequenzen B weiterhin in einzelsträngiger Form vorliegen, das heißt, hybridisiert sind, oder ob sie sich zumindest zum Teil mit den Test-Polynucleotidsequenzen B zu Doppelsträngen verbunden haben, das heißt, nunmehr in denaturierter Form vorliegen. Eine eventuelle Intensitätsänderung ist dabei ein Maß für die Absorption, welche in der in Kontakt mit dem Testmedium befindlichen Probe auftritt und ist damit äquivalent zum Imaginärteil des komplexen Brechungsindexes der in Kontakt mit dem Testmedium befindlichen Probe.

Alternativ zur Erfassung der Absorption und damit des Imaginärteils des komplexen Brechungsindexes kann auch der Realteil des imaginären Brechungsindexes der in Kontakt mit dem Testmedium befindlichen Probe erfasst werden, insbesondere auch eine Änderung des Realteils des komplexen Brechungsindexes. Auch eine solche Änderung des Realteils des komplexen Brechungsindexes kann zum Nachweis dafür verwendet werden, ob die in der Probe enthaltenen Polynucleotide X im wesentlichen in hybridisierter oder in denaturierter Form vorliegen, nachdem die Probe in Kontakt mit dem Testmedium gebracht wurde. Weitere Details hierzu ergeben sich aus den Ausführungsbeispielen.

Alternativ zur Verwendung der gepulsten Terahertzspektroskopie PTS, hier vorzugsweise zeitaufgelöst, kann die in Schritt c) des erfindungsgemäßen Verfahrens durchzuführende Ermittlung der mindestens einen Komponente des komplexen Brechungsindexes oder der dazu äquivalenten Größe auch mittels Wechselwirkung der Probe mit Terahertzstrahlung durchgeführt werden, die schmalbandig ist und frequenzaufgelöst detektiert wird. Insbesondere kommen hierzu beispielsweise Transmissionsmessungen kontinuierlicher oder quasikontinuierlicher, das heißt schmalbandiger Terahertzstrahlung durch die in Kontakt mit dem Testmedium befindliche Probe hindurch in Frage. Weiterhin kann mittels Diffraktionsmessungen direkt der Realteil des komplexen Brechungsindexes der in Kontakt mit dem Testmedium befindlichen Probe vermessen werden. Schließlich kommen auch alle aus dem Stand der Technik bekannten Verfahren in Frage, welche zur Erfassung mindestens einer Komponente des komplexen Brechungsindexes oder einer dazu äquivalenten Größe mittels schmalbandiger Terahertzstrahlung geeignet sind.

Wird die verwendete schmalbandige Terahertzstrahlung frequenzaufgelöst detektiert, so ist eine Übertragung der aus der Optik bekannten Streumethoden wie Ramanstreuung bzw. Brillouinstreuung an Molekülen im Terahertzbereich möglich. Auch mittels entsprechender Streuversuche können die Komponenten des komplexen Brechungsindexes oder dazu äquivalente Größen in dem im Hauptanspruch genannten Frequenzintervall ermittelt werden.

Zur Durchführung des erfindungsgemäßen Verfahrens unter Verwendung der gepulsten Terahertzspektroskopie PTS sind wiederum mehrere Möglichkeiten denkbar, in welcher Weise die zu untersuchende Probe, welche sich in Kontakt mit dem Testmedium befindet, in Wechselwirkung mit der gepulsten Terahertzstrahlung gebracht werden kann.

In einer ersten Weiterbildung des erfindungsgemäßen Verfahrens propagiert die gepulste Terahertzstrahlung nach ihrer Erzeugung mittels einer dazu geeigneten THz-Quelle frei, bis sie in Wechselwirkung mit der in Kontakt mit dem Testmedium befindlichen Probe gebracht wird. Nach der Wechselwirkung propagiert die gepulste Terahertzstrahlung wiederum frei, bis sie zu einem THz-Detektor gelangt. Dieses Verfahren wird im folgenden als "Freistrahlanordnung" bezeichnet.

Alternativ hierzu kann eine sogenannte "Nahfeldanordnung" realisiert werden. Diese beruht auf der Erkenntnis, dass eine besonders hohe Intensität der für das erfindungsgemäße Verfahren erforderlichen Terahertzstrahlung im Nahfeld von speziell hierzu geeigneten Nahfeldemittern erzielt werden kann. In einer weiteren Weiterbildung des erfindungsgemäßen Verfahrens wird daher die erforderliche Terahertzstrahlung mittels eines Nahfeldemitters in unmittelbarer Nähe der in Kontakt mit dem Testmedium befindlichen Probe generiert. Nach der Wechselwirkung der Terahertzstrahlung mit der im Kontakt mit dem Testmedium befindlichen Probe kann die Terahertzstrahlung mittels konventioneller Strahlführungsmethoden zu einem geeigneten Detektor geführt werden.

Alternativ hierzu ist auch eine komplementäre Anordnung möglich, bei der die Terahertzstrahlung mittels eines konventionellen Terahertzemitters erzeugt wird und mittels konventioneller Strahlführungsvorrichtungen wie z. B. metallischer Spiegel zur in Kontakt mit dem Testmedium befindlichen Probe geführt werden. Nach der Wechselwirkung der Terahertzstrahlung mit der in Kontakt mit dem Testmedium befindlichen Probe kann die Terahertzstrahlung mittels eines geeigneten Terahertznahfelddetektors detektiert und analysiert werden.

Beide Ausbildungen der Nahfeldanordnung bieten den Vorteil, dass eine hohe Ortsauflösung erzielt werden kann, die besonders dann von Vorteil ist, wenn die nachzuweisenden hybridisierten oder denaturierten Polynucleotidsequenzen auf ein Substrat aufgebracht sind, insbesondere wenn die Test-Polynucleotidsequenz B auf einem Substrat fixiert ist. Analog zu den aus dem Stand der Technik bekannten Array-Nachweisverfahren zur gleichzeitigen Analyse einer Probe auf verschiedene Polynucleotidsequenzen A, A'... einem Substrat kann die Probe anhand der zusätzlich verfügbaren Ortsinformation auf das Vorhandensein einer Vielzahl verschiedener Polynucleotidsequenzen A gleichzeitig untersucht werden.

In einer weiteren vorteilhaften Weiterbildung des erfindungsgemäßen Verfahrens propagiert die zur Spektroskopie erforderliche Terahertzstrahlung vor, während oder nach der Wechselwirkung mit der in Verbindung mit dem Testmedium befindlichen Probe auf oder in einem Wellenleiter. Hierdurch ergibt sich eine sehr hohe Effizienz der Strahlführung. Weiterhin können mittels vorbekannter Strukturierungsverfahren, beispielsweise aus dem Bereich der Halbleitertechnik, miniarturisierte Vorrichtungen realisiert werden, die zur Durchführung des erfindungsgemäßen Verfahrens geeignet sind.

Schließlich ergeben sich besondere Vorteile bei der Durchführung des erfindungsgemäßen Verfahrens, wenn die Wellenleiterstruktur im Bereich der in Verbindung mit dem Testmedium befindlichen Probe so ausgebildet ist, dass sich aufgrund veränderter Eigenschaften der Terahertzstrahlung in diesem Bereich eine erhöhte Sensitivität des erfindungsgemäßen Verfahrens ergibt. Insbesondere kann die Wellenleiterstruktur hierzu im Bereich der in Verbindung mit dem Testmedium befindlichen Probe eine Resonatorstruktur aufweisen, welche die Feldstärke der Terahertzstrahlung lokal erhöht. Weiterhin ist es möglich, mittels geeigneter Wellenleiterstrukturen, beispielsweise mittels eines Frequenzfilters, die Wellenleitereigenschaften dergestalt zu beeinflussen, dass sie frequenzabhängig werden. Hierdurch kann beispielsweise eine scharfe Kante in der frequenzabhängigen Transmission durch die Wellenleiterstruktur erzeugt werden, welche durch zumindest eine Komponente des komplexen Brechungsindexes der in Verbindung mit dem Testmedium stehenden Probe oder einer hierzu äquivalenten Größe beeinflusst werden kann. Insbesondere kann beispielsweise durch eine Änderung des komplexen Brechungsindexes, beispielsweise durch eine Änderung der Absorption, eine Verschiebung der Frequenzkante auftreten.

In Analogie zu den aus dem Stand der Technik vorbekannten Nachweisverfahren für Polynucleotidsequenzen, welche auf der Markierung der nachzuweisenden Polynucleotidsequenz A mittels Fluoreszenzfarbstoffen beruhen, hat es sich als besonders vorteilhaft zur Durchführung des erfindungsgemäßen Verfahrens erwiesen, wenn die im Testmedium enthaltenen komplementären Test-Polynucleotidsequenzen B auf oder in einem Substrat fixiert sind. Auf diese Weise kann eine hohe Konzentration der nachzuweisenden Polynucleotidsequenzen A im denaturierten Zustand an derjenigen Oberfläche erzielt werden, an welcher die komplementären Test-Polynucleotidsequenzen B fixiert sind. Insbesondere in Verbindung mit oberflächen- oder grenzflächensensitiven Nachweisverfahren für das Vorliegen denaturierter Polynucleotidsequenzen, welche nach dem erfindungsgemäßen Verfahren ausgeführt werden, ist eine solche erhöhte Konzentration an der Grenzfläche vorteilhaft.

In einer weiteren Verbesserung des erfindungsgemäßen Verfahrens werden nach Verfahrensschritt b) die in der Probe enthaltenen, aber nicht an die komplementären Test-Polynucleotidsequenzen B gebundenen Polynucleotidsequenzen X vor der Ermittlung der zumindest einen Komponente des komplexen Brechungsindexes oder der dazu äquivalenten Größe vom Testmedium entfernt. Insbesondere kann dies mittels Abwaschen des in Kontakt mit der Probe befindlichen Testmediums geschehen. Dieser zusätzliche Zwischenschritt vermeidet Probleme, welche dadurch verursacht werden können, dass in der Probe selbst nicht nur die nachzuweisende einzelsträngige Polynucleotidsequenz A enthalten sein kann, sondern auch die dazu komplementäre Polynucleotidsequenz B in Form von Einzelsträngen. Ein solches gleichzeitiges Vorliegen der komplementären Polynucleotidsequenzen A und B kann beispielsweise durch vorangegangene Verfahrensschritte zur Hybridisierung von in der Probe enthaltenen Polynucleotidsequenzen X und nachfolgender ungenügender Trennung der Einzelstränge verursacht werden.

Vorzugsweise wird dabei das Testmedium dergestalt abgewaschen, dass die in der Probe enthaltenen Polynucleotidsequenzen X, welche an die vorzugsweise auf einem Substrat fixierten komplementären Polynucleotidsequenzen B gebunden sind, haften bleiben, während der Rest der Probe entfernt wird.

In einer weiteren vorteilhaften Ausgestaltung des erfindungsgemäßen Verfahrens wird eine Mehrzahl N verschiedener Polynucleotidsequenzen A (1), ...., A (N) in der Probe gleichzeitig nachgewiesen. Hierzu wird eine Mehrzahl N von Testmedien präpariert, die jeweils eine zu den nachzuweisenden Polynucleotidsequenzen A (1), ...., A (N) komplementäre Test-Polynucleotidsequenz B (1), ...., B (N) enthält. Dabei werden die Testmedien an voneinander verschiedenen Orten auf oder in einem Substrat fixiert. Mittels der Verfahrensschritte b) und c) wird nunmehr für jedes einzelne, in Verbindung mit der Probe befindliche Testmedium zumindest eine Komponente des komplexen Brechungsindexes oder einer dazu äquivalenten Größe ermittelt. Dies kann mittels lokal auflösender Nachweisverfahren realisiert werden. Insbesondere können hierzu die Wechselwirkung mit fokussierter Terahertzstrahlung oder die Verwendung von Terahertz-Nahfeldemittern oder-Nahfeldprobern herangezogen werden.

Weitere Vorteile und Merkmale des erfindungsgemäßen Verfahrens ergeben sich aus den Unteransprüchen sowie den nun folgenden Ausführungsbeispielen, welche nicht einschränkend zu verstehen sind und anhand der Zeichnung erläutert werden. In dieser zeigen:

Fig. 1: eine schematische Darstellung einer Vorrichtung zur Durchführung des erfindungsgemäßen Verfahrens in einer Freistrahlanordnung,

Fig. 2: eine schematische Darstellung einer Vorrichtung zur Durchführung des erfindungsgemäßen Verfahrens in einer ersten Nahfeldanordnung,

Fig. 3: eine schematische Darstellung einer Vorrichtung zur Ausführung des erfindungsgemäßen Verfahrens in einer zweiten Nahfeldanordnung,

Fig. 4: eine schematische Darstellung einer weiteren Vorrichtung zur Durchführung des erfindungsgemäßen Verfahrens, einen Wellenleiter für die Terahertzstrahlung umfassend,

Fig. 5a: eine Vorrichtung gemäß Figur 4, wobei in den Wellenleiter eine Resonatorstruktur integriert ist,

Fig. 5b: eine Vorrichtung gemäß Figur 4, wobei in den Wellenleiter eine Filterstruktur integriert ist,

Fig. 6: ein zweidimensionales Array von Wellenleiterstrukturen beispielsweise gemäß Figur 4,

Fig. 7a: Ergebnisse einer Transmissionsmessung mittels zeitaufgelöster Terahertzspektroskopie durch eine Probe, welche eine nachzuweisende Polynucleotidsequenz in denaturierter Form enthält,

Fig. 7b: Ergebnisse einer Transmissionsmessung durch eine Probe, welche die nachzuweisende Polynucleotidsequenz in hybridisierter Form enthält,

Fig. 8: den frequenzabhängigen Verlauf des Realteils des komplexen Brechungsindexes einer Probe, die nachzuweisende Polynucleotidsequenz in hybridisierter oder denaturierter Form enthält und

Fig. 9: die Ergebnisse einer Transmissionsmessung durch eine planare Wellenleiterstruktur.

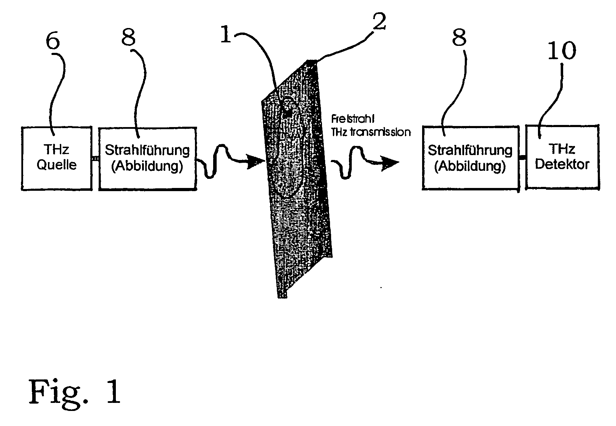

Fig. 1 zeigt eine erste Anordnung zur Ausführung des erfindungsgemäßen Verfahrens. Als Probe 1 wird eine wässrige Lösung verwendet, in der verschiedene, in Form von Einzelsträngen vorliegende, d.h. denaturierte Polynucleotidsequenzen X gelöst sind. Nachzuweisen ist, ob in der Probe 1 eine bestimmte einzelsträngige Polynucleotidsequenz A enthalten ist. Dabei kann A entweder mit einer der Polynucleotidsequenzen X identisch sein oder in dieser in identischer Form enthalten sein, d.h. entweder

$$X = A$$

oder

$$X = Y Z A V Z$$

wobei V, Y, Z beliebige andere Polynucleotidsequenzen darstellen können.

Die Probe 1 ist auf die Oberfläche eines Substrats 2 aufgebracht, welches aus einem Saphir-Plättchen mit einer Dicke von ca. 1mm besteht. Auf der Substratoberfläche 2 sind einzelsträngige Test-Polynucleotidsequenzen B fixiert, welche zur nachzuweisenden Polynucleotidsequenz A komplementär sind. Das Substrat 2 bildet gemeinsam mit den darauf fixierten Test-Polynucleotidsequenzen B das Testmedium 4.

Die in der Probe 1 enthaltenen Polynucleotidsequenzen X gelangen in der wässrigen Lösung an die Oberfläche des Substrats 2 und damit der darauf fixierten Test-Polynucleotidsequenzen B. Ist eine der Polynucleotidsequenzen X mit A identisch oder A in X in identischer Form enthalten, so kann diese Polynucleotidsequenz X an eine Test-Polynucleotidsequenz B binden, d.h. in Verbindung mit B eine hybridisierte (also doppelsträngige) Polynucleotidsequenz X-B bilden. Da eine Bindung zwischen X und B nur auftritt, wenn A mit X identisch ist oder A in X als Sequenzabschnitt enthalten ist, liegt somit die nachzuweisende Polynucleotidsequenz A in hybridisierter Form in der in Kontakt mit dem Testmedium 4 befindlichen Probe 1 vor. Da B an der Oberfläche des Substrats 4 fixiert ist, ist auch die doppelsträngige Polynucleotidsequenz X-B an der Oberfläche fixiert. Im Laufe der Zeit nimmt daher die Konzentration der die gesuchte Polynucleotidsequenz A enthaltenden oder mit dieser identischen einzelsträngigen Polynucleotidsequenzen X in der Probelösung ab, gleichzeitig steigt die Konzentration der doppelsträngigen Polynucleotidsequenzen X-B an der Substratoberfläche. An der Substratoberfläche wird damit eine starke Anreicherung von A gegenüber der Probe 1 erreicht.

Mittels geeigneter Verfahren wird nunmehr unter Verwendung elektromagnetischer Strahlung im Frequenzbereich zwischen 0,1 THz und 20 THz zumindest eine Komponente des komplexen Brechungsindexes der in Kontakt mit dem Testmedium 4 befindlichen Probe 1 oder eine dazu äquivalente Größe bestimmt.

Um den Einfluss eventuell noch in der wässrigen Probe 1 enthaltener weiterer Polynucleotidsequenzen auf diese Verfahren zu minimieren, ist es vorteilhaft, das Substrat 2 abzuwaschen und zu trocken, so dass möglichst nur noch die an die Test-Polynucleotidsequenzen B gebundenen Polynucleotidsequenzen X aus der Probe auf der Substratoberfläche verbleiben, während der Rest der Probe 1 einschließlich des Lösungsmittels, hier Wasser, entfernt wird. Es entsteht an der Substratoberfläche ein dünner Film, der im wesentlichen nur noch aus doppelsträngigen Polynucleotidsequenzen X-B und eventuell Einlagerungen von Wasser besteht.

Mittels einer THz-Quelle 6 wird nunmehr breitbandige gepulste elektromagnetische Strahlung erzeugt, deren Frequenz im Frequenzintervall zwischen 0,1 THz und 20 THZ liegt. Typische Pulsdauern liegen zwischen 0,01 Picosekunden (ps) und 10 ps, die Pulswiederholraten liegen im Bereich von mehreren 10 bis 100 MHz.

Die gepulste THz-Strahlung wird mittels geeigneter Mittel zur Strahlführung 8 zum Substrat 2 geführt und auf dieses abgebildet. Insbesondere wird derjenige Bereich des Substrats 2 bestrahlt, in dem sich die doppelsträngigen Polynucleotidsequenzen X-B an der Substratoberfläche angereichert haben, falls die gesuchte Polynucleotidsequenz A in der Probe 1 enthalten war. Zur Strahlführung kommen beispielsweise metallische Hohlspiegel in Frage.

Die in Kontakt mit dem Testmedium 4 befindliche Probe 1, hier also der fragliche Bereich der Substratoberfläche, insbesondere die hierin enthaltenen, entweder denaturierten Test-Polynucleotidsequenzen B oder hybridisierten doppelsträngigen Polynucleotidsequenzen X-B, wird der THz-Strahlung ausgesetzt und wechselwirkt mit dieser. Hieraus ergeben sich Änderungen der eingestrahlten THz-Strahlung, welche einen Rückschluss auf das Vorliegen oder die Abwesenheit von doppelsträngigen Polynucleotidsequenzen X-B zulassen und damit auf das Vorliegen der nachzuweisenden Polynucleotidsequenz A in der Probe 1.

Nach der Wechselwirkung mit der in Kontakt mit dem Testmedium 4 befindlichen Probe 1 wird die durch das Substrat 2 transmittierte THz-Strahlung wiederum mittels geeigneter Mittel zur Strahlführung 8 von der Probe 1 zu einem THz-Detektor 10 geführt und dort analysiert. Figur 1 zeigt daher eine Freistrahl-Anordnung zur Durchführung des erfindungsgemäßen Verfahrens, bei der die THz-Strahlung vor und nach der Wechselwirkung mit der in Kontakt mit dem Testmedium 4 befindlichen Probe 1 frei propagiert.

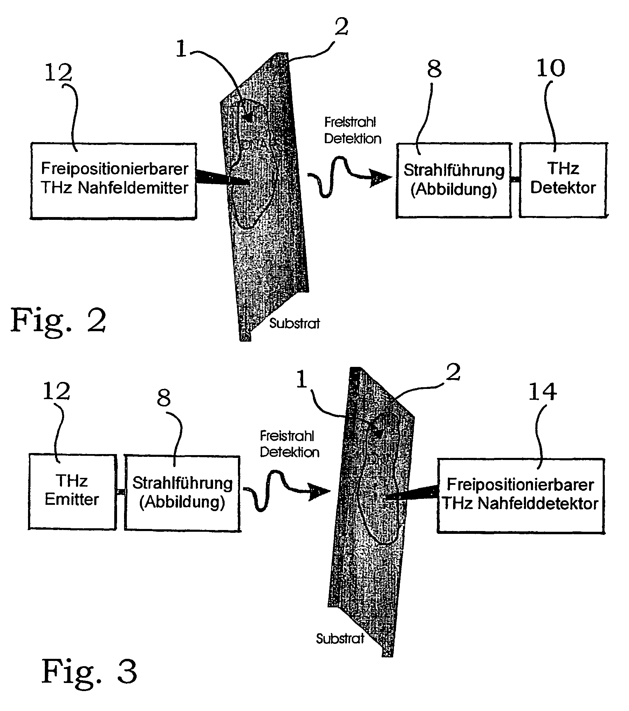

Eine alternative Anordnung ist aus Figur 2 ersichtlich. Bei gleicher Anordnung der Probe 1 auf dem Substrat 2 wird die THz-Strahlung in unmittelbarer Nähe der Substratoberfläche, insbesondere desjenigen Bereichs, in dem die Test-Polynucleotidsequenzen B fixiert sind, mittels eines relativ zur Substratoberfläche frei positionierbaren THz-Nahfeldemitters 12 erzeugt. Ein solcher THz-Nahfeldemitter 12 kann eine z.B. in Halbleitertechnik ausgeführte miniaturisierte THz-Quelle sein, welche insbesondere mit einer THz-Antenne zur gerichteten Abstrahlung von THz-Strahlung kombiniert sein kann. Eine THz-Quelle 6 mit Abmessungen, die deutlich unter der Wellenlänge der THz-Strahlung liegen, ein sogenannter THz-Nahfeldemitter 12 kann beispielsweise mit Techniken realisiert werden, die aus der nahfeldoptischen Mikroskopie bekannt sind. Hierzu wird auf die Vielzahl von Veröffentlichungen zum Thema "scanning nearfield optical microscopy" (kurz: SNOM) verwiesen.

Grundsätzlich wird in der Nahfeldoptik angestrebt, auch bei optischen Nachweisverfahren ein Auflösungsvermögen unterhalb der Wellenlänge des verwendeten Lichts zu erzielen. Dies ist mittels spezieller optischer Nahfeldemitter möglich, die in ihrem optischen Nahfeld, welches sich nur einige 10 Nanometer von der Emissionsfläche des Nahfeldemitters erstreckt, eine Konzentration der abgestrahlten Intensität in einem Strahlungsbündel erzielen, welches einen Durchmesser aufweist, der um zumindest eine Größenordnung unterhalb der Wellenlänge der emittierten Strahlung liegt. Bringt man einen Nahfeldemitter in einem solchen Abstand vor eine Probe, dass die Probe im Nahfeld des Nahfeldemitters liegt, so ist eine hoch-ortsaufgelöste Abfrage optischer Eigenschaften der Probe möglich.

Wird im Rahmen des erfindungsgemäßen Verfahrens in analoger Weise ein THz-Nahfeldemitter 12 verwendet, so können die "THz-optischen" Eigenschaften der hier zu untersuchenden Probe 1 in gleicher Weise mit hoher Ortsauflösung erfasst werden. Insbesondere kann der komplexe Brechungsindex der Probe 1, zumindest eine Komponente hiervon, oder eine hierzu äquivalente Größe mit hoher Ortsauflösung abgefragt werden.

Die vom THz-Nahfeldemitter 12 emittierte THz-Strahlung trifft auf die mit dem Testmedium 4 in Kontakt befindliche Probe 1 und wechselwirkt mit dieser. Nach der Wechselwirkung mit der Probe 1 wird die transmittierte THz-Strahlung wiederum mittels geeigneter Mittel zur Strahlführung 8 zu einem THz-Detektor 10 geführt und von diesem detektiert.

Im Vergleich zu der in Figur 1 gezeigten Anordnung kann mit der in Figur 2 gezeigten Anordnung daher eine deutlich erhöhte räumliche Auflösung erzielt werden, welche Vorteile bei der Miniaturisierung der zur Durchführung des erfindungsgemäßen Verfahrens erforderlichen Vorrichtung, sowie bei der simultanen Durchführung des Verfahrens an einer Vielzahl von Proben 1 oder mittels einer Vielzahl von Testmedien 4 bietet.

Eine vergleichbar hohe Auflösung bietet auch die aus Fig. 3 ersichtliche Vorrichtung zur Durchführung des Verfahrens. Erzeugung und Strahlführung der THz-Strahlung zur Probe 1 sowie Wechselwirkung der THz-Strahlung mit der Probe entspricht derjenigen in Fig. 1. Jedoch wird die durch die in Kontakt mit dem Testmedium 4 befindliche Probe 1 transmittierte THz-Strahlung nunmehr mittels eines frei positionierbaren THz-Nahfelddetektors 14 mit hoher Ortsauflösung detektiert. Dabei weist der THz-Nahfelddetektor 14 analoge "nahfeldoptische" Eigenschaften auf wie der Nahfeldemitter 12 in Fig. 2 und ist im Nahfeld der Probe angeordnet.

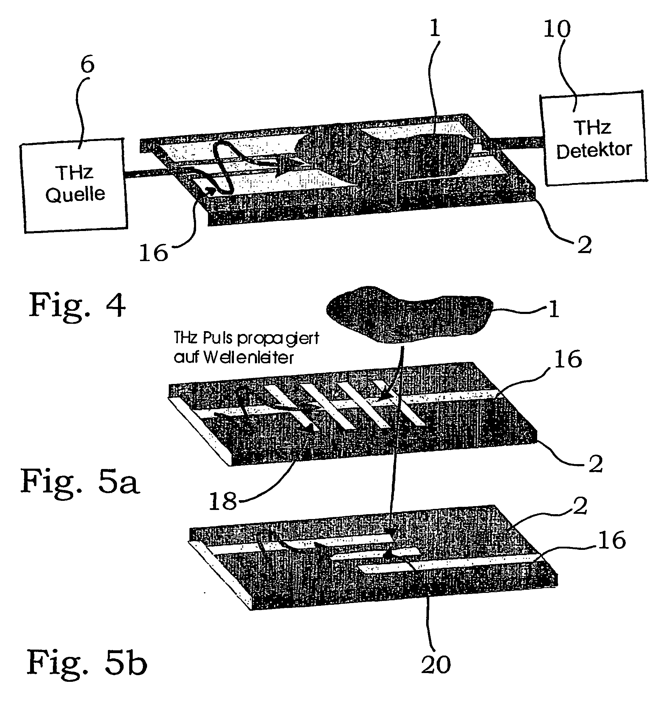

Fig. 4 zeigt einen alternativen Zugang zur Durchführung des erfindungsgemäßen Verfahrens auf. In das Substrat 4, auf welches die zu untersuchende Probe 1 aufgetropft ist, ist eine Wellenleiterstruktur 16 für die THz-Strahlung integriert, längs der die THz-Strahlung propagieren kann. Die Probe 1 ist auf die Oberfläche der Wellenleiterstruktur 16 aufgebracht. Da das Feld der THz-Strahlung als evaneszente Welle auch aus der Wellenleiterstruktur 16 senkrecht zu deren Oberfläche herausreicht, kann die längs des Wellenleiters 16 propagierende THz-Strahlung mit der Probe 1 zumindest im oberflächennahen Bereich der Wellenleiterstruktur 16 wechselwirken. Tatsächlich ist die Feldstärke der evaneszenten Welle an der Oberfläche der Wellenleiterstruktur 16 sogar erhöht gegenüber frei im Raum propagierender THz-Strahlung.

Nach der Wechselwirkung der THz-Strahlung mit der zu untersuchenden Probe 1 wird die längs der Wellenleiterstruktur transmittierte THz-Strahlung mittels eines geeigneten THz-Detektors 10 detektiert. Dieser kann entweder analog zu dem aus Fig. 1 ersichtlichen THz-Detektor 10 für frei propagierende THz-Strahlung ausgebildet sein, oder alternativ z.B. in Halbleitertechnik in die Wellenleiterstruktur 16 integriert sein.

Fig. 5a zeigt eine Wellenleiterstruktur 16, in welche zusätzlich eine Resonatorstruktur 18 integriert ist, welche resonant für die verwendete THz-Strahlung ist. Im Bereich der Resonatorstruktur 18 ergibt sich eine stark erhöhte Feldstärke der in der Wellenleiterstruktur propagierenden THz-Strahlung. Bringt man die Probe 1 im Bereich der Resonatorstruktur 18 auf den Wellenleiter auf, so ist dort die Feldstärke der evaneszenten Welle nochmals erhöht, woraus sich eine erhöhte Sensitivität der Anordnung aufgrund eines verbesserten Signal-zu-Rausch-Verhältnisses ergibt.

Fig. 5b zeigt eine weitere Wellenleiterstruktur, in welche ein frequenzselektives Element 20, z.B. ein Kurzpassfilter für die längs der Wellenleiterstruktur propagierende THz-Strahlung integriert ist. Dieses frequenzselektive Element weist bezüglich seiner Transmissivität für THz-Strahlung zumindest eine scharfe Abschneidekante auf. Die genaue Lage dieser Abschneidekante wird durch die Eigenschaften des frequenzselektiven Elements 20 bestimmt und kann unter anderem auch stark von den Oberflächeneigenschaften des Substrats, in oder auf welchem das frequenzselektive Element 20 ausgebildet ist, abhängig sein. Veränderungen an der Substratoberfläche, z.B. des Realteils des komplexen Brechungsindexes einer oberflächennahen Schicht durch Anlagerung von Polynucleotidsequenzen X an die dort fixierten Test-Polynucleotidsequenzen B, kann zu einer starken Veränderung der Eigenschaften des frequenzselektiven Elements 20 führen, insbesondere zu einer Verschiebung der Abschneidekante. Diese Verschiebung kann wiederum ausgenutzt werden, um zumindest eine Komponente des komplexen Brechungsindexes der in Kontakt mit dem Testmedium 4 befindlichen Probe oder eine dazu äquivalente Größe, hier z.B. eine veränderte Transmission der THz-Strahlung durch das frequenzselektive Element 20, zu erfassen.

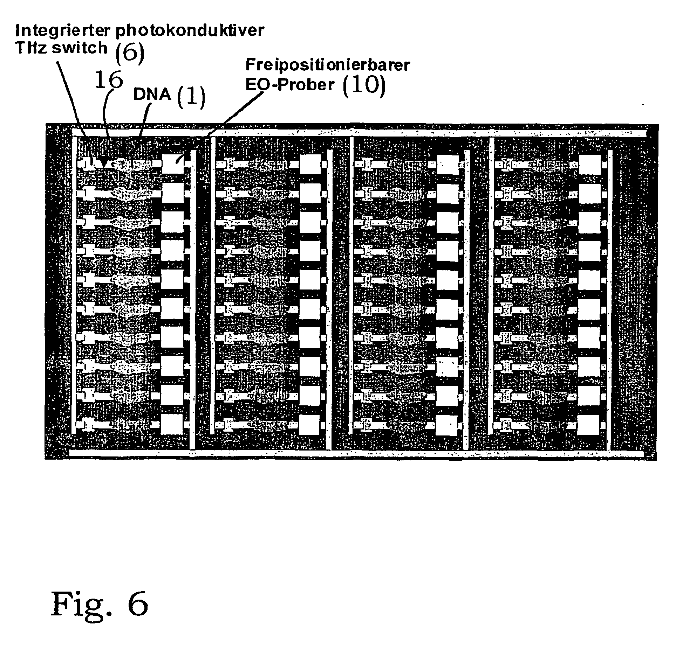

Schließlich ist aus Figur 6 ein 2-d Array von Wellenleiterstrukturen 16 ersichtlich, welche den aus Fig. 4 ersichtlichen Aufbau aufweisen. Als THz-Detektoren 10 werden elektrooptische Kristalle verwendet ("EO-Prober"), deren durch einfallende THz-Strahlung beeinflusste optischen Eigenschaften mittels geeigneter Verfahren zeitaufgelöst erfasst werden. Zur optimalen Einkopplung der längs der Wellenleiterstruktur 16 propagierenden THz-Strahlung können diese EO-Prober frei auf der Oberfläche des Substrats 2 positioniert werden. Das aus Fig. 6 ersichtliche Array ermöglicht eine parallele Durchführung des erfindungsgemäßen Verfahrens an einer Vielzahl verschiedener Proben 1 oder mittels einer Vielzahl verschiedener Testmedien 4.

Alle voranstehend beschriebenen Anordnungen beruhen auf der Detektion und Analyse transmittierter THz-Strahlung. Selbstverständlich sind alle Anordnungen in analoger Weise auch in Reflexion möglich, ebenso kann bei der Wechselwirkung mit der Probe 1 gestreute THz-Strahlung detektiert und analysiert werden.

Versuchsreihe 1:

In einer ersten Versuchsreihe wurde des Vorliegen einer bestimmten (DNA-) Polynucleotidsequenz A in denaturierter oder hybridisierter Form in einer Probe untersucht. Als nachzuweisende Polynucleotidsequenz wurde hierzu der Vector "pcDNA3" (bezogen von Invitrogen, Carlsbad, CA, USA) ausgewählt, der eine Größe von 5,4kB aufweist. Der Vektor wurde in E.coli amplifiziert und unter Verwendung des Nucleobond-PC500-Systems (bezogen von Macherey-Nagel, Düren, BRD) gereinigt. Die Konzentration der (DNA-) Polynucleotidsequenz A, d.h. des Vektors pcDNA3, wurde in einer ersten Probe auf 17 $\mu$g/$\mu$l und in einer zweiten Probe auf 5 $\mu$g/$\mu$l in zweifach destilliertem Wasser eingestellt.

Die in der Probe in doppelsträngiger Form vorliegenden (DNA-) Polynucleotidsequenzen wurden in einem Teil der Probelösungen denaturiert, indem die Temperatur der Probelösung über einen Zeitraum von 5 Minuten auf 95°C erhöht wurde. Der Denaturierungsprozess wurde durch Abkühlung mittels Eis schockartig gestoppt, um eine Renaturierung der nunmehr in einzelsträngiger Form vorliegenden (DNA-) Polynucleotide zu unterdrücken.

In derjenigen Lösung, in der der Denaturierungsprozess abrupt gestoppt wurde, lag somit das nachzuweisende Polynucleotid A in Form separierter Einzelstränge vor. In der Terminologie des Hauptanspruchs enthielt diese Lösung daher gleichzeitig das nachzuweisende (einzelsträngige) Polynucleotid A und das dazu komplementäre Test-Polynucleotid B in Form der voneinander getrennten Einzelstränge des Vektors pcDNA3.

In derjenigen Lösung, in der keine Denaturierung des Vektors pcDNA3 vorgenommen wurde, lag in der Terminologie des Hauptanspruchs somit das nachzuweisende (einzelsträngige) Polynucleotid A in an das komplementäre Test-Polynucleotid B gebundener Form in der Probelösung vor.

In beiden Fällen stellte somit die Probelösung die Probe 1 im Sinne des Hauptanspruchs dar, die sich in Kontakt mit dem Testmedium 4 befand.

In einer ersten Versuchsreihe wurden Untersuchungen an den Polynucleotidproben in Freistrahlanordnung durchgeführt. Hierzu wurden 2 $\mu$l große Tropfen der Probelösung, welche Polynucleotide in denaturierter oder hybridisierter Form in einer Konzentration von 17 $\mu$g/$\mu$l enthielten, auf SaphirSubstrate aufgebracht und 48 Stunden getrocknet, um die Restfeuchte auf unter 1% zu reduzieren. Der Wassergehalt der Proben hat sich in Untersuchungen von [Markelz 00] als relevant für den Brechungsindex der Proben im THz-Bereich herausgestellt. Nach dem Eintrocknen der Tropfen ergaben sich dünne Filme mit einem Durchmesser von etwa 2 mm und einer Dicke von etwa 30 $\mu$m.

An den präparierten dünnen Filmen wurden Transmissionsmessungen bei 300 Kelvin in trockener Atmosphäre durchgeführt. Hierzu wurde ein Ständard-Aufbau zur zeitaufgelösten gepulsten Terahertzspektroskopie verwendet, wie er aus beispielsweise aus [Smith 88] bekannt ist. Als Lichtquelle zur Erzeugung der THz-Pulse wurde ein kommerzielles Femtosekunden-Ti: Saphir-Lasersystem verwendet, welches Pulse mit einer Pulsdauer von etwa 100 fs im nahen Infraroten erzeugte.

Abwechselnd wurden jeweils mindestens 10 unabhängige Referenzmessungen an unbeschichteten Substraten und Messungen an mit der zu untersuchenden Probe beschichteten Substraten durchgeführt. Um den Einfluss unvermeidlicher experimenteller Schwankungen zu minimieren, wurden alle Messungen an mehreren verschiedenen Proben wiederholt.

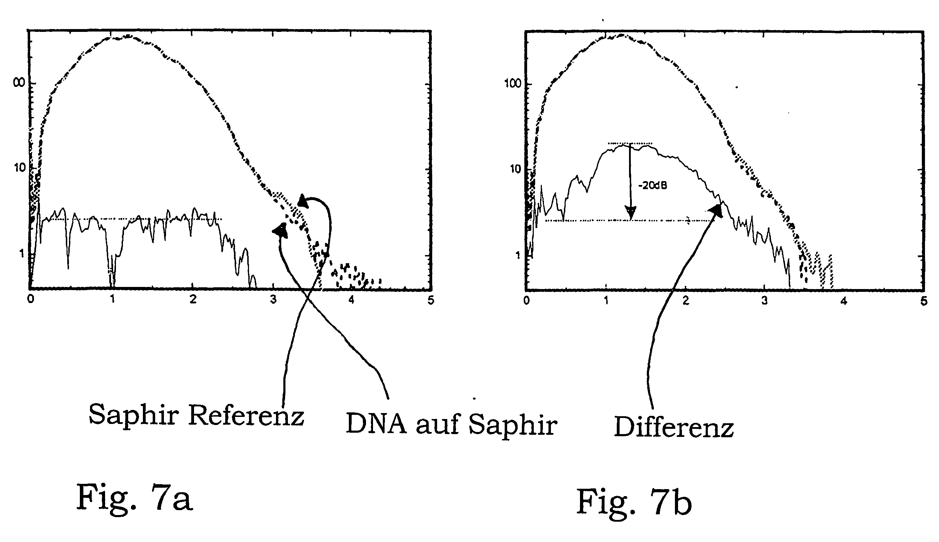

Fig. 7a zeigt ein frequenzaufgelöstes THz-Transmissionsspektrum, welches aus einer Fouriertransformation der zeitaufgelöst erfassten Pulse nach der Wechselwirkung mit Proben gewonnen wurde, die die nachzuweisende Polynucleotidsequenz A, d.h. den Vektor pcDNA3 in denaturierter, also einzelsträngiger Form enthielten. Dabei wurden die Transmissionsmessungen in Freistrahlanordnung unter senkrechtem Einfall auf die Probe durchgeführt.

Fig. 7b zeigt dagegen das zeitaufgelöste THz-Transmissionsspektrum, welches in der gleichen Versuchsanordnung an den Proben gewonnen wurde, die die nachzuweisende Polynucleotidsequenz A in hybridisierter, also doppelsträngiger Form enthielten.

Beide Figuren 7a und 7b zeigen den Verlauf der Transmissionsspektren einerseits der unbedeckten Saphirsubstrate (gepunktete Linie) und andererseits der mit den dünnen Filmen beschichteten Saphirsubstrate (gestrichelte Linie). Als dünne durchgezogene Linie ist jeweils die komplexe Differenz der Transmissionsspektren des unbedeckten und des bedeckten Substrats gezeigt.

Für beide Proben sind die Transmissionsspektren von unbedecktem und mit der jeweiligen Probe beschichtetem Substrat sehr ähnlich, was durch die geringe Dicke der Probe bedingt ist. Die Differenzspektren (dünne durchgezogene Linie) beider Proben zeigen jedoch einen Verlauf, der stark davon abhängt, ob die nachzuweisende Polynucleotidsequenz A, d.h. der Vektor pcDNA3, in Form von Einzelsträngen, d.h. denaturiert, oder in Form von Doppelsträngen, d.h. hybridisiert, vorliegt. Die dünne gepunktete Linie in Fig. 7a zeigt den Mittelwert der frequenzabhängigen komplexen Differenz zwischen unbedecktem Saphirsubstrat und mit der Probe beschichtetem Saphirsubstrat.

Im Fall derjenigen Probe, welche die nachzuweisende Polynucleotidsequenz A in denaturierter Form enthielt, ergibt sich nur eine geringe komplexe Differenz, welche praktisch frequenzunabhängig ist, wie aus Fig. 7a ersichtlich ist. Im Gegensatz dazu ergibt sich im Fall derjenigen Probe, welche die nachzuweisende Polynucleotidsequenz A in hybridisierter Form enthielt, eine deutlich erhöhte komplexe Differenz, welche eine starke Frequenzabhängigkeit zeigt. Aus dem Vergleich beider komplexer Differenzen ergibt sich, dass die komplexe Differenz im Fall der hybridisierten Probe um ca. 20 dB über der komplexen Differenz im Fall der denaturierten Probe liegt.

Eine Unterscheidung zwischen hybridisierter und denaturierter Polynucleotidsequenz A oder dazu komplementärer Test-Polynucleotidsequenz B im Sinne des Hauptanspruchs ist daher z.B. bereits anhand der Unterschiede zwischen den komplexen Differenzen zwischen einem Testmedium 4, welches nur die komplementäre Test-Polynucleotidsequenz B enthält, und einem in Kontakt mit der Probe 1 befindlichen Testmedium 4 möglich. Enthält die Probe 1 die nachzuweisende Polynucleotidsequenz A, so liegt A in hybridisierter Form vor, nämlich gebunden an die komplementäre Test-Polynucleotidsequenz B. Enthält die Probe 1 dagegen die nachzuweisende Polynucleotidsequenz A nicht, so liegt nur die denaturierte Test-Polynucleotidsequenz B vor. Werden wie vorstehend beschrieben die komplexen Differenzen der Transmissionsspektren durch einerseits das Testmedium 4 alleine und andererseits durch das in Kontakt mit der Probe 1 befindliche Testmedium 4 miteinander verglichen, so ist anhand des Verlaufs der komplexen Differenzen eine Unterscheidung möglich, ob die komplementäre Test-Polynucleotidsequenz B in denaturierter oder in hybridisierter Form vorliegt. Im ersten Fall enthält die Probe 1 die nachzuweisende Polynucleotidsequenz nicht, im zweiten Fall ist A in der Probe enthalten. Damit ist der komplexe Brechungsunterschied eine zum komplexen Brechungsindex äquivalente Größe im Sinne des Hauptanspruchs.

Weiterhin können sowohl der Realteil als auch der Imaginärteil des komplexen Brechungsindexes der Probe als Nachweiskriterium für den Bindungszustand der Test-Polynucleotidsequenz B und damit das Vorliegen der nachzuweisenden Polynucleotidsequenz A in der Probe 1 herangezogen werden. Aus den frequenzabhängigen Transmissionsspektren der Figuren 7a und 7b können unter Verwendung der Fresnel-Formeln direkt die komplexen Brechungsindices der Proben berechnet werden. Der Realteil des komplexen Brechungsindexes ist dabei ein Maß für die Dispersion der in Kontakt mit dem Testmedium 4 befindlichen Probe.

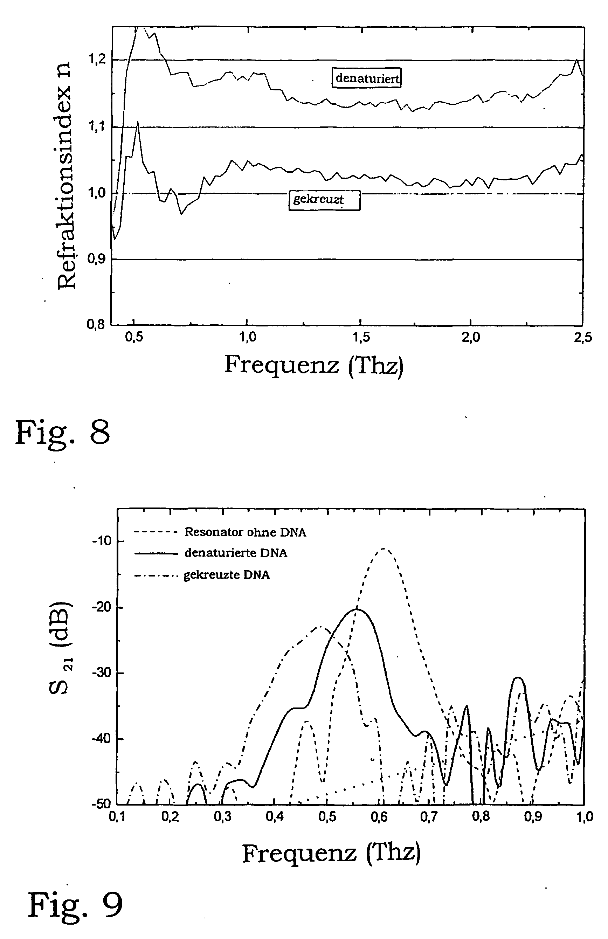

Die untere Kurve in Fig. 8 zeigt den Verlauf des Realteils der komplexen Brechungsindices derjenigen Proben, welche die nachzuweisende Polynucleotidsequenz A in einzelsträngiger, d.h. denaturierter Form enthielten. Die obere Kurve in Fig. 8 zeigt dagegen den Verlauf des Realteils des komplexen Brechungsindices derjenigen Proben, welche die nachzuweisende Polynucleotidsequenz A in doppelsträngiger, d.h. hybridisierter Form enthielten. Beide Kurven sind klar voneinander zu unterscheiden, da sie einen Unterschied von ca. $\Delta n \geq 0{,}1$ im gezeigten Frequenzintervall aufweisen. Damit ist anhand einer Bestimmung des Unterschieds zwischen den Realteilen der komplexen Brechungsindices zweier Proben, von denen die eine nur die Test-Polynucleotidsequenz B enthält, welche somit in denaturierter Form vorliegt, und die andere die Test-Polynucleotidsequenz B in Kontakt mit der Probe 1 enthält, somit also die Test-Polynucleotidsequenz B in hybridisierter Form enthält, falls die nachzuweisende Polynucleotidsequenz A in der Probe 1 enthalten ist, ein Nachweis der gesuchten Polynucleotidsequenz A in der Probe 1 möglich.

Analoge Aussagen sind auch anhand des Imaginärteils des komplexen Brechungsindexes der in Kontakt mit dem Testmedium 4 befindlichen Probe 1 möglich, welche die Absorption in der Probe charakterisiert. Dies ist hier jedoch nicht explizit gezeigt. Es hat sich gezeigt, dass der Realteil des komplexen Brechungsindexes eine geringere Anfälligkeit für Verunreinigungen aufweist und daher i.a. eine erhöhte Genauigkeit des erfindungsgemäßen Verfahrens bei Verwendung des Realteils des komplexen Brechungsindexes der Probe als Nachweiskriterium erzielt werden kann.

Die Tatsache, dass die Probe, welche die nachzuweisende Polynucleotidsequenz in hybridisierter Form enthielt, einen deutlich erhöhten Brechungsindex aufweist, deutet in Übereinstimmung mit den bereits zitierten theoretischen Vorarbeiten von [Saxena 89] und [Zhuang 90] darauf hin, dass in doppelsträngigen Polynucleotidsequenzen Anregungszustände existieren, deren Anregungsenergien in dem im Hauptanspruch genannten Frequenzbereich der THz-Strahlung liegen, wobei diese Anregungszustände in einzelsträngigen Polynucleotidsequenzen nicht vorhanden oder stark unterdrückt sind. Eine Abfrage dieser Anregungszustände ist daher ein geeignetes Mittel zur Unterscheidung zwischen denaturierten oder hybridisierten Polynucleotidsequenzen, insbesondere der Test-Polynucleotidsequenz B, im Rahmen des erfindungsgemäßen Verfahrens.

Es sei an dieser Stelle darauf hingewiesen, dass auch eine reine Absorptionsmessung in dem im Hauptanspruch genannten Frequenzintervall eine Möglichkeit zur Ermittlung zumindest einer Komponente des komplexen Brechungsindexes oder einer dazu äquivalenten Größe der in Kontakt mit dem Testmedium 4 befindlichen Probe 1 darstellt, da die in der Probe auftretende Absorption direkt mit dem Imaginärteil des komplexen Brechungsindexes verknüpft ist.

<u>Versuchsreihe 2:</u>

In einer zweiten Versuchsreihe wurden die Proben nicht in einer Freistrahlanordnung, sondern einer Wellenleiteranordnung untersucht. Dabei wurde von der Verschiebung der Transmissionscharakteristik eines in eine Wellenleiterstruktur integrierten Resonators durch Änderung der dielektrischen Eigenschaften einer Deckschicht auf den Resonator Gebrauch gemacht.

Hierzu wurde eine planare Wellenleiterstruktur realisiert, in die ein Resonator mit einer Resonanzfrequenz von ca. 600 GHz integriert war. Fig. 9 zeigt den frequenzabhängigen Verlauf der die Transmission durch den Resonator kennzeichnenden Größe $S_{21}$ im Frequenzintervall zwischen 0,1 und 1 THz. Die dünne gestrichelte Kurve zeigt den Verlauf für einen unbedeckten Resonator.

Wird auf ein Tropfen der Probelösung auf die planare Wellenleiterstruktur im Bereich des Resonators aufgebracht und dort eingetrocknet, so wird der Resonator an seiner Oberfläche mit einer dünnen Schicht dielektrischen Materials beschichtet, nämlich mit den in der Probelösung enthaltenen, entweder denaturierten oder hybridisierten Polynucleotidsequenzen A (pcDNA3). Diese dielektrische Deckschicht verändert die Transmissionscharakteristik des Resonators.

Die dicke durchgezogene Kurve in Fig. 9 zeigt den Verlauf von $S_{21}$ für eine Deckschicht bestehend aus einer eingetrockneten Probe (Konzentration: 5μg/μl, Tropfenvolumen: 0,6μl, Trocknungszeit: 2h), welche die Polynucleotidsequenz A in denaturierter Form enthielt. Die dünne strichpunktierte Kurve in Fig. 9 zeigt dagegen den Verlauf von $S_{21}$ für eine Deckschicht bestehend aus einer eingetrockneten Probe, welche die Polynucleotidsequenz A in hybridisierter Form enthielt. Man erkennt deutliche Unterschiede zwischen beiden Kurven. Um die Unterschiede zu charakterisieren, kann z.B. die Verschiebung Δν des Transmissionsmaximums bzw. die Veränderung ΔT der Höhe des Transmissionsmaximums herangezogen werden. Im gezeigten Beispiel ergeben sich im Vergleich zum unbedeckten Resonator:

1. für die Probe, welche die nachzuweisende Polynucleotidsequenz pcDNA3 in denaturierter Form enthielt: Δν = -45 GHz, ΔT = -9 dB, und

2. für die Probe, welche die nachzuweisende Polynucleotidsequenz pcDNA3 in hybridisierter Form enthielt: Δν = -120 GHz, ΔT = -12 dB.

Die Verschiebung und die Veränderung der Höhe des Transmissionsmaximums des Resonators sind dabei direkt mit dem komplexen Brechungsindex der auf den Resonator aufgebrachten Proben korreliert. Daher sind beide Größen als zu den Komponenten des komplexen Brechungsindexes der in Kontakt mit dem Testmedium 4 befindlichen Probe 1 äquivalente Größen im Sinne des Hauptanspruchs zu verstehen.

Der Bindungszustand einer auf den Resonator aufgebrachten Test-Polynucleotidsequenz B (hybridisiert vs. denaturiert) kann daher beispielsweise anhand der Größen Δν und ΔT des Resonators bestimmt werden. Anhand dieser Größen kann daher unterschieden werden, ob in der Probe 1 die nachzuweisende Polynucleotidsequenz vorhanden war oder nicht.

Im allgemeinen wird in einer Wellenleiteranordnung, insbesondere unter Verwendung sensitivitätssteigernder frequenzabhängiger Strukturen wie Filter oder Resonatoren, eine höhere Nachweisempfindlichkeit erzielt werden können als in einer Freistrahlanordnung. Die Wellenleiteranordnung eignet sich daher besonders für die Durchführung des erfindungsgemäßen Verfahrens im Maßstab eines medizinischen Labors.

Abschließend wird festgestellt, dass die hier vorgestellten Ausführungsbeispiele auf der Generation von THz-Strahlung unter Verwendung eines Femtosekunden-Lasersystems beruhen. Künftige Fortschritte vor allem in der Halbleitertechnik lassen jedoch erwarten, dass bereits in naher Zukunft effiziente Quellen für THz-Strahlung im relevanten Frequenzbereich zur Verfügung stehen werden, die eine kommerzielle Anwendung des Verfahrens ermöglichen werden.

# EP 1 301 774 B1

Selbstverständlich kann das erfindungsgemäße Verfahren auf Polynucleotidsequenzen (praktisch) beliebiger Länge angewendet werden, insbesondere auch auf kurzkettige Polynucleotidsequenzen mit weniger als 20 Basen(-paaren), die in der Literatur oftmals als Oligonucleotidsequenzen bezeichnet werden.

**Literaturverzeichnis:**

| [Katzenellenbogen 92] | N. Katzenellenbogen et al., in: Betroni (Ed.), Ultra-Wideband, Short Wavelength Electromagnetics, Plenum, N.Y. 1992 |
|---|---|
| [Wittlin 95] | A. Wittlin et al., Phys. Rev. A 34 (1986) 493 |
| [Cai 98] | Y. Cai et al., Appl. Phy. Lett. 72 (1998) 444 |
| [Smith 88] | P.R. Smith, D.H. Auston and M.C. Nuss, IEEE J. Quantum Electron. 24, 255, 1988. |
| [Nuss 98] | M.C. Nuss and J. Orenstein, "THz time-domain spectroscopy" in Millimeter and Sub-Millimeter Waves, ed. by G. Gruener (Springer--Verlag, Heidelberg, 1998). |
| [Haring 99] | P. Haring Bolivar, "Coherent THz spectroscopy", in *Semiconductor Quantum Optoelectronics: From Quantum Physics to Smart Devices* ed. by A. Miller, M. Ebrahimzahdeh and D.M. Finlayson, ISBN 0-7503-0628-9, (Institute of Physics Publishing, Bristol, 1999), pp. 151-192. |
| [Dahl 98] | C.Dahl, P. Goy and J. Kotthaus, "Magneto-optical millimeter-wave spectroscopy" in Millimeter and Sub-Millimeter Waves, ed. by G. Gruener (Springer-Verlag, Heidelberg, 1998). |
| [Saxena 89] | V.K. Saxena and L.L. Van Zandt, Phys. Rev. A **40**, 6134 (1989). |
| [Zhuang 90] | W. Zhuang, Y. Feng, E.W. Prohofsky, Phys. Rev. A **41**, 7033 (1990) |
| [Markelz 00] | A.G. Markelz, A. Roitberg, E.J. Heilweil, Chem. Phys. Lett. **320**, 42 (2000) |
| [Smith 88] | P.R. Smith, D.H. Auston and M.C. Nuss, IEEE J. Quantum Electron. **QE 24**, 255, 1988. |

## Patentansprüche

**1.** Verfahren zum Nachweis einer Polynucleotidsequenz A in einer Probe, welche eine Mehrzahl identischer oder verschiedener Polynucleotidsequenzen X als Einzelstränge enthält, und wobei die Polynucleotidsequenz A mit einer der Polynucleotidsequenzen X identisch sein kann oder als Sequenzabschnitt in einer der Polynucleotidsequenzen X enthalten sein kann, über die Abfrage des Bindungszustandes der in der Probe enthaltenen Polynucleotidsequenzen X an eine bekannte, zur Polynucleotidsequenz A komplementäre Test-Polynucleotidsequenz B, die folgenden Verfahrensschritte aufweisend:

a) Präparation eines Testmediums, welches zur nachzuweisenden Polynucleotidsequenz A komplementäre Test-Polynucleotidsequenzen B als Einzelstränge enthält,

b) In Kontakt bringen der Probe mit dem Testmedium durch Ein- oder Aufbringen der Probe in oder auf das Testmedium, so dass die in der Probe enthaltenen Einzelstränge der Polynucleotidsequenzen X an die im Testmedium enthaltenen komplementären Test-Polynucleotidsequenzen B binden können, **dadurch gekennzeichnet, dass** zum Nachweis einer Bindung von Polynucleotidsequenzen X an Test-Polynucleotidsequenzen B folgender Schritt c) ausgeführt wird:

c) Überprüfung, ob im Frequenzbereich zwischen 0,1 Terahertz (THz) und 20 THz durch das in Kontakt bringen der Probe mit dem Testmedium gemäß Schritt b) eine Änderung zumindest einer Komponente des komplexen Brechungsindexes oder einer äquivalenten Größe der Probe aufgetreten ist, die mit der Ausbildung doppelsträngiger Polynucleotidsequenzen aus den einzelsträngigen Polynucleotidsequenzen X und den im Testmedium enthaltenen einzelsträngigen komplementären Test-Polynucleotidsequenzen B korreliert ist.

2. Verfahren nach Anspruch 1, **dadurch gekennzeichnet, dass** die Überprüfung gemäß Schritt c) mittels Terahertz-Spektroskopie (TS) durchgeführt wird.

3. Verfahren nach Anspruch 2, **dadurch gekennzeichnet, dass** die verwendete Terahertz-Strahlung gepulst ist ("gepulste Terahertz-Spektroskopie" PTS), wobei die Pulse eine Pulsdauer zwischen 0,05 Picosekunden (ps) und 10 ps aufweisen und zeitaufgelöst detektiert werden.

4. Verfahren nach Anspruch 3, **dadurch gekennzeichnet, dass** die verwendete Terahertz-Strahlung frequenzaufgelöst detektiert wird.

5. Verfahren nach Anspruch 1, **dadurch gekennzeichnet, dass** eine Mehrzahl N verschiedener Polynucleotidsequenzen A(1),..., A(N) in der Probe nachgewiesen werden soll, wozu eine Mehrzahl N von Testmedien präpariert wird, die jeweils eine zu den nachzuweisenden Polynucleotidsequenzen A(1),..., A(N) komplementäre Test-Polynucleotidsequenz B(1), B(N) enthält, wobei die Testmedien an voneinander verschiedenen Orten auf oder in einem Substrat fixiert sind, und die Verfahrensschritte b) und c) für jedes einzelne, in Verbindung mit der Probe befindliche Testmedium durchgeführt werden.

6. Vorrichtung zur Durchführung des Verfahrens gemäß Anspruch 1, die folgenden Komponenten aufweisend:

   a. eine Terahertz-Quelle (6) für elektromagnetische Strahlung,
   b. eine Wellenleiterstruktur (16), längs der die von der Terahertz-Quelle erzeugte elektromagnetische Strahlung propagieren kann, wobei auf eine Oberfläche der Wellenleiterstruktur (16) das Testmedium mit den komplementären Polynucleotidsequenzen B aufgebracht ist, und
   c. einen Terahertz-Detektor (10) zur Detektion der längs der Wellenleiterstruktur (16) transmittierten oder von der Probe (1) gestreuten elektromagnetischen Strahlung.

7. Vorrichtung gemäß Anspruch 6, **dadurch gekennzeichnet, dass** die Wellenleiterstruktur (16) ein im Bereich des aufgebrachten Testmediums angeordnetes frequenzselektives Element (20) umfasst, welches die Feldstärke der Terahertz-Strahlung lokal erhöht.

8. Vorrichtung zur Durchführung des Verfahrens gemäß Anspruch 1, die folgenden Komponenten aufweisend:

   a. eine Terahertz-Quelle (6) für elektromagnetische Strahlung,
   b. einen Terahertz-Detektor (10) zur Detektion der durch die mit dem Testmedium in Kontakt befindliche Probe (1) transmittierten oder der von der Probe (1) reflektierten oder gestreuten elektromagnetischen Strahlung, und
   c. ein Mittel zur Strahlführung (8), das dazu vorgesehen ist, die durch die Probe (1) transmittierte oder der von der Probe (1) reflektierte oder gestreute elektromagnetischen Strahlung zum Terahertz-Detektor (10) zu führen,
   **dadurch gekennzeichnet, dass**
   d. die Terahertz-Quelle (10) als relativ zur Probe (1) positionierbarer Terahertz-Nahfeldemitter (12) ausgebildet ist.

9. Vorrichtung zur Durchführung des Verfahrens gemäß Anspruch 1, die folgenden Komponenten aufweisend:

   a. eine Terahertz-Quelle (6) für elektromagnetische Strahlung,
   b. ein Mittel zur Strahlführung (8), das dazu vorgesehen ist, die von der Terahertz-Quelle (6) erzeugte elektromagnetische Strahlung zu der mit dem Testmedium in Kontakt befindlichen Probe (1) zu führen, und
   c. einen Terahertz-Detektor (10) zur Detektion der durch die Probe (1) transmittierten oder der von der Probe (1) reflektierten oder gestreuten elektromagnetischen Strahlung,
   **dadurch gekennzeichnet, dass**
   d. der Terahertz-Detektor (10) als relativ zur Probe (1) positionierbarer Terahertz-Nahfelddetektor (14) ausgebildet ist.

**Claims**

1. A method of evidencing the presence of a polynucleotide sequence A in a sample containing a plurality of identical or different polynucleotide sequences X in the form of individual strands, where said polynucleotide sequence A may be identical to one of the polynucleotide sequences X or contained in one of the polynucleotide sequences X in the form of a sequence portion, by determining the binding status of the polynucleotide sequences X contained in the sample from a known test polynucleotide sequence B, which is complementary to the polynucleotide sequence A, said method comprising the following steps:

a) preparing a test medium which contains, in the form of individual strands, test polynucleotide sequences B which are complementary to the polynucleotide sequence A to be evidenced,

b) bringing the sample into contact with said test medium by introducing the sample into, or applying it on, said test medium, so as to allow the individual strands of the polynucleotide sequences X contained in the sample to bind to the complementary test polynucleotide sequences B contained in said test medium,

**characterized in that**, for evidencing the binding of polynucleotide sequences X to test polynucleotide sequences B, the following step c) is carried out:

c) checking whether, in the frequency range from between 0.1 terahertz (THz) and 20 THz, contacting the sample with the test medium in accordance with step b) has caused a change to occur in at least one component of the complex index of refraction or an equivalent variable of the sample that is correlated with the formation of double-stranded polynucleotide sequences from the single-stranded polynucleotide sequences X and the single-stranded complementary test polynucleotide sequences B contained in said test medium.

2. The method as set forth in claim 1, **characterized in that** the checking of step c) is carried out using Terahertz Spectroscopy (TS).

3. The method as set forth in claim 2, **characterized in that** the Terahertz radiation used is pulsed ("pulsed Terahertz Spectroscopy" PTS), the pulses having a pulse duration ranging between 0.05 picoseconds (ps) and 10 ps and being detected by a time resolved technique.

4. The method as set forth in claim 3, **characterized in that** the terahertz radiation used is detected by a frequency-resolved technique.

5. The method as set forth in claim 1, **characterized in that** the presence of a plurality N of different polynucleotide sequences A(1), ..., A(N) is to be evidenced in the sample, a plurality N of test media each of which contains a test polynucleotide sequence B(1), ..., B(N) that is complementary to the polynucleotide sequences A(1), ..., A(N) to be evidenced being prepared for this purpose, said test media being fixed onto or in a substrate at different locations and the method steps b) and c) being carried out for every single one of the test media connected with the sample.

6. A device for carrying out the method as set forth in claim 1, said device comprising the following components:

a. a terahertz source (6) for electromagnetic radiation,

b. a wave guide structure (16) along which the electromagnetic radiation generated by the terahertz source is allowed to propagate, the test medium containing the complementary polynucleotide sequences B being applied to a surface of the wave guide structure (16) and

c. a terahertz detector (10) for detecting the electromagnetic radiation transmitted along the wave guide structure (16) or scattered by the sample (1).

7. The device as set forth in claim 6, **characterized in that** the wave guide structure (16) includes a frequency-selective element (20) that is disposed in the region of the test medium applied and locally increases the field intensity of the terahertz radiation.

8. A device for carrying out the method as set forth in claim 1, said device comprising the following components:

a. a terahertz source (6) for electromagnetic radiation,

b. a terahertz detector (10) for detecting the electromagnetic radiation transmitted through the sample (1) contacting the test medium or reflected or scattered by the sample (1) and

c. a means for guiding the beam (8) that is provided for guiding the electromagnetic radiation transmitted through the sample (1) or reflected or scattered by the sample (1) to the terahertz detector (10),

**characterized in that**

d. the terahertz source (10) is configured to be a terahertz near field emitter (12).

**9.** A device for carrying out the method as set forth in claim 1, said device comprising the following components:

a. a terahertz source (6) for electromagnetic radiation,
b. a means for guiding the beam (8) that is provided for guiding the electromagnetic radiation generated by the terahertz source (6) to the sample (1) contacting the test medium, and
c. a terahertz detector (10) for detecting the electromagnetic radiation transmitted through the sample (1) or reflected or scattered by the sample (1),
**characterized in that**
d. the terahertz source (10) is configured to be a terahertz near field detector (14).


**Revendications**

**1.** Procédé pour prouver la présence d'une séquence polynucléotidique A dans un échantillon contenant une pluralité de séquences polynucléotidiques X identiques ou différentes sous forme de simples brins, la séquence polynucléotidique A pouvant être identique à une des séquences polynucléotidiques X ou pouvant être contenue sous forme de portion de séquence dans une des séquences polynucléotidiques X, par détermination de l'état de liaison des séquences polynucléotidiques X contenues dans l'échantillon à partir d'une séquence polynucléotidique d'essai B complémentaire de la séquence polynucléotidique A, du type comportant les étapes suivantes :

a) on prépare un support d'essai contenant des séquences polynucléotidiques d'essai B sous forme de simples brins complémentaires de la séquence polynucléotidique A dont il y a lieu de prouver la présence,
b) on met l'échantillon en contact avec le support d'essai en introduisant l'échantillon dans le support d'essai ou en l'appliquant sur celui-ci de manière à permettre aux simples brins des séquences polynucléotidiques X contenus dans l'échantillon de se lier aux séquences polynucléotidiques d'essai B complémentaires contenues dans le support d'essai,
**caractérisé en ce que**, pour prouver la liaison de séquences polynucléotidiques X avec des séquences polynucléotidiques d'essai B, on passe à l'étape c) suivante :
c) on vérifie si, dans la plage de fréquences comprise entre 0,1 térahertz (THz) et 20 THz, le fait de mettre l'échantillon en contact avec le support d'essai conformément à l'étape b) a provoqué le changement, corrélé à la formation de séquences polynucléotidiques double brin à partir des séquences polynucléotidiques X simple brin et des séquences polynucléotidiques d'essai B simple brin complémentaires contenues dans le support d'essai, d'au moins un composant de l'indice de réfraction complexe ou d'une grandeur équivalente de l'échantillon.

**2.** Procédé selon la revendication 1, **caractérisé en ce que** la vérification conformément à l'étape c) est effectuée moyennant la spectroscopie térahertz (TS).

**3.** Procédé selon la revendication 2, **caractérisé en ce que** le rayonnement térahertz utilisé est impulsionnel (« spectroscopie térahertz impulsionnelle », PTS), les impulsions ayant une durée comprise entre 0,05 picosecondes (ps) et 10 ps et étant détectées par une détection résolue en temps.

**4.** Procédé selon la revendication 3, **caractérisé en ce que** le rayonnement tétrahertz utilisé est détecté par une technique résolue en fréquence.

**5.** Procédé selon la revendication 1, **caractérisé en ce que** la présence d'une pluralité N de séquences polynucléotidiques A(1), ..., A(N) différentes doit être prouvée dans l'échantillon, une pluralité N de supports d'essai contenant chacun une séquence polynucléotidique d'essai B(1), ..., B(N) complémentaire aux séquences polynucléotidiques A(1), ..., A(N) dont il y a lieu de prouver la présence étant préparée à cet effet, les supports d'essai étant fixés à différents endroits sur ou dans un substrat, et les étapes b) et c) du procédé étant effectuées pour chacun des supports d'essai se trouvant relié à l'échantillon.

**6.** Dispositif destiné à mettre en oeuvre le procédé selon la revendication 1, du type comportant les composants suivants :

a. une source térahertz (6) de rayonnement électromagnétique,
b. une structure de guides d'onde (16) le long de laquelle le rayonnement électromagnétique généré par la source térahertz peut se propager, le support d'essai contenant les séquences polynucléotidiques B complémentaires étant appliqué sur une surface de la structure de guides d'onde (16), et

c. un détecteur térahertz (10) destiné à détecter le rayonnement électromagnétique transmis le long de la structure de guides d'ondes (16) ou diffusé par l'échantillon (1).

7. Dispositif selon la revendication 6, **caractérisé en ce que** la structure de guides d'ondes (16) comprend un élément sélecteur de fréquences (20) qui est disposé à la hauteur du support d'essai appliqué et qui augmente localement l'intensité de champ du rayonnement térahertz.

8. Dispositif pour la mise en oeuvre du procédé selon la revendication 1, du type comportant les composants suivants :

a. une source térahertz (6) de rayonnement électromagnétique,

b. un détecteur térahertz (10) destiné à détecter le rayonnement électromagnétique transmis à travers l'échantillon (1) se trouvant en contact avec le support d'essai ou reflété ou diffusé par l'échantillon (1), et

c. un moyen pour guider le faisceau de rayons (8) qui est destiné à guider le rayonnement électromagnétique transmis à travers l'échantillon (1) ou réfléchi ou diffusé par l'échantillon (1) vers le détecteur térahertz (10),

**caractérisé en ce que**

d. la source térahertz (10) est conformée en forme d'émetteur térahertz en champ proche (12).

9. Dispositif pour la mise en oeuvre du procédé selon la revendication 1, du type comportant les composants suivants :

a. une source térahertz (6) de rayonnement électromagnétique,

b. un moyen pour guider le faisceau de rayons (8) qui est destiné à guider le rayonnement électromagnétique généré par la source térahertz (6) vers l'échantillon (1) se trouvant en contact avec le support d'essai, et

c. un détecteur térahertz (10) destiné à détecter le rayonnement électromagnétique transmis à travers l'échantillon (1) ou reflété ou diffusé par l'échantillon (1),

**caractérisé en ce que**

d. le détecteur térahertz (10) est conformé en forme de détecteur térahertz en champ proche (14).

**Fig. 1**

2

1

12

Freistrahl
Detektion

8

10

Freipositionierbarer
THz Nahfeldemitter

Strahlführung
(Abbildung)

THz
Detektor

Fig. 2

Substrat

1

2

12

8

Freistrahl
Detektion

14

THz
Emitter

Strahlführung
(Abbildung)

Freipositionierbarer
THz Nahfelddetektor

Fig. 3

Substrat

**Fig. 4**

THz Puls propagiert
auf Wellenleiter

**Fig. 5a**

**Fig. 5b**

Integrierter photokonduktiver
THz switch (6)

16

DNA (1)

Freipositionierbarer
EO-Prober (10)

Fig. 6

Fig. 7a    Fig. 7b

Fig. 8

Fig. 9